# FASCICULE DE BREVET EUROPEEN

(11) **EP 3 123 233 B1**
(45) Date de publication et mention de la délivrance du brevet: **02.12.2020**
(21) Numéro de dépôt: 15714867.7
(22) Date de dépôt: 12.03.2015
(51) Int. Cl.: G02B 27/18, A61B 5/00, A61F 9/08, A61N 1/05, G03B 21/00, G06N 3/04, H04N 5/369, G06K 9/60, G06T 7/20

(54) **DISPOSITIF DE VISUALISATION D'UNE SEQUENCE D'IMAGES ET SYSTEME DE VISUALISATION D'UNE SCENE**
VORRICHTUNG ZUR ANZEIGE EINER BILDSEQUENZ UND SYSTEM ZUR ANZEIGE EINER SZENE
DEVICE FOR DISPLAYING AN IMAGE SEQUENCE AND SYSTEM FOR DISPLAYING A SCENE

(30) Priorité: 26.03.2014 FR 1452558
(43) Date de publication de la demande: 01.02.2017
(73) Titulaire: SORBONNE UNIVERSITE, 75006 Paris (FR); CNRS Centre National de la Recherche Scientifique, 75016 Paris (FR)
(72) Inventeur: BENOSMAN, Ryad, F-93500 Pantin (FR); CHENEGROS, Guillaume, F-78190 Trappes (FR); PICAUD, Serge, F-77210 Avon (FR); IENG, Siohoi, F-93100 Montreuil (FR); SAHEL, José-Alain, 75013 Paris (FR)
(74) Mandataire: Plasseraud IP
(86) Numéro de dépôt international: PCT/FR2015/050615
(87) Numéro de publication internationale: WO 2015/145017

(56) Documents cités:
- FR-A1- 2 975 251
- US-A1- 2014 009 591
- HENRI LORACH ET AL: "Paper;Artificial retina: the multichannel processing of the mammalian retina achieved with a neuromorphic asynchronous light acquisition device;Artificial retina: the multichannel processing of the mammalian retina achieved with a neuromorphic asynchronous light acquisition device", JOURNAL OF NEURAL ENGINEERING, INSTITUTE OF PHYSICS PUBLISHING, BRISTOL, GB, vol. 9, no. 6, 17 octobre 2012 (2012-10-17), page 66004, XP020234329, ISSN: 1741-2552, DOI: 10.1088/1741-2560/9/6/066004
- POSCH C: "Bio-inspired vision", JOURNAL OF INSTRUMENTATION, INSTITUTE OF PHYSICS PUBLISHING, BRISTOL, GB, vol. 7, no. 1, 12 janvier 2012 (2012-01-12), page C01054, XP020217109, ISSN: 1748-0221, DOI: 10.1088/1748-0221/7/01/C01054
- CHRISTOPH POSCH ET AL: "Biomimetic frame-free HDR camera with event-driven PWM image/video sensor and full-custom address-event processor", BIOMEDICAL CIRCUITS AND SYSTEMS CONFERENCE (BIOCAS), 2010 IEEE, IEEE, 3 novembre 2010 (2010-11-03), pages 254-257, XP031899745, DOI: 10.1109/BIOCAS.2010.5709619 ISBN: 978-1-4244-7269-7
- PIATKOWSKA EWA ET AL: "Asynchronous Stereo Vision for Event-Driven Dynamic Stereo Sensor Using an Adaptive Cooperative Approach", 2013 IEEE INTERNATIONAL CONFERENCE ON COMPUTER VISION WORKSHOPS, IEEE, 2 décembre 2013 (2013-12-02), pages 45-50, XP032575709, DOI: 10.1109/ICCVW.2013.13

## Description

La présente invention se rapporte à des dispositifs de visualisation d'informations visuelles à partir d'une information asynchrone.

Des dispositifs ayant la forme de lunettes permettant à un utilisateur de visualiser une scène captée par une caméra sur un écran miniature, fixé sur la monture ou agencé sur un verre, sont commercialisés depuis quelques années. La caméra est en général fixée sur la monture des lunettes de manière à capter une scène dans le champ de vision de l'utilisateur, par exemple pour des applications de réalité augmentée.

Il existe par ailleurs des dispositifs d'aide à la vision destinés à des utilisateurs équipés d'un implant visuel répondant à des stimulations électriques. Certains de ces dispositifs se présentent sous la forme de lunettes sur lesquelles sont montées une caméra qui produit un flux vidéo envoyé vers une unité de traitement vidéo qui encode ce flux pour générer un signal de stimulation électrique, et une bobine externe pour transmettre par induction électromagnétique le signal à une bobine interne reliée à un implant rétinien.

Ces dispositifs existants ne peuvent cependant pas être utilisés par des personnes équipées d'un implant visuel à photodiodes, ou bien des personnes ayant bénéficié d'un traitement optogénétique. En effet, les implants ou les procédés de traitement optogénétiques requièrent des signaux de stimulation lumineuse d'intensité beaucoup plus élevée que celle de la lumière ambiante. Un implant d'aide à la vision placé sous l'œil, comprenant typiquement une électrode autour de laquelle sont disposées une à trois photodiodes, ne fonctionnera efficacement que si ces photodiodes reçoivent une lumière sept fois plus puissante que celle de la lumière ambiante, de manière à ce que les photodiodes puissent émettre un stimulus. De même, les traitements optogénétiques actuels ne sont pleinement efficaces que si l'œil traité reçoit des signaux lumineux ayant une longueur d'onde spécifique et une intensité lumineuse allant de deux à sept fois celle de la lumière ambiante. Les puissances lumineuses requises sont donc tellement élevées que l'utilisation des procédés d'affichage mis en œuvre sur les dispositifs classiques, à ces niveaux de puissance, provoquerait des lésions sur les organes visuels des utilisateurs.

Il existe ainsi un besoin pour des dispositifs de visualisation d'une scène ne présentant pas les inconvénients des méthodes classiques exposés ci-dessus. En particulier, un premier besoin est de fournir des dispositifs de visualisation d'une scène qu'il soit possible d'utiliser pour des applications dans le domaine de l'aide à la vision. Un autre besoin est de fournir des dispositifs de visualisation d'une scène qui soient compatibles avec une utilisation par des personnes équipées d'un implant visuel à photodiodes et/ou des personnes ayant bénéficié d'un traitement optogénétique.

Selon un premier aspect, il est proposé un dispositif de visualisation d'une séquence d'images sous forme d'une matrice de pixels, comprenant une unité de commande couplée de manière opérationnelle à un projecteur, l'unité de commande comprenant une interface d'entrée configurée pour recevoir de l'information asynchrone représentant, pour chaque pixel de la matrice, des évènements concernant le pixel et un processeur configuré pour commander l'activation de chaque pixel de la matrice à des instants déterminés par des événements respectifs indiqués par l'information asynchrone pour ledit pixel. Le projecteur du dispositif de visualisation proposé est agencé sur un support de manière à illuminer des photorécepteurs de l'œil lors de l'utilisation du dispositif, et configuré pour projeter un flux lumineux correspondant aux pixels activés par l'unité de commande.

Les évènements concernant le pixel peuvent selon le mode de réalisation correspondre à des variations de lumière pour le pixel, à la détection d'une forme d'intérêt ou à la détection d'une primitive, et plus généralement à tout type d'information asynchrone pour le pixel.

L'utilisation d'une information asynchrone représentant des évènements pour générer des commandes pour l'activation des pixels d'une matrice présente de nombreux avantages. Ceux-ci résultent notamment du fait que ces signaux ne sont pas échantillonnés dans le temps selon une fréquence d'horloge prédéfinie, comme l'horloge des trames dans un signal vidéo classique. Ils fournissent ce qu'on appelle une représentation adresse-événement (AER, « address-event représentation ») d'une scène à visualiser. A chaque pixel correspond une séquence de signal basé sur évènement (« event based »).

Les procédés d'acquisition ou de synthèse d'une séquence d'images par trames présentent l'inconvénient de produire des données avec une forte redondance, due au fait que chaque trame représente un grand nombre de pixels d'une image si ce n'est une image entière, et que l'ensemble de ces pixels pour lesquels l'information ne change pas d'une image à l'autre, génère des redondances dans les données représentant la séquence d'images. Cette redondance ne peut être qu'en partie supprimée par un encodage de compression d'un signal vidéo classique. A l'inverse, les signaux asynchrones permettent d'obtenir une représentation très compacte de données relatives à une séquence d'images, du fait que ces données, représentant des événements pour un pixel (et non pas pour tous les pixels d'une matrice ou un grand nombre d'entre eux), ne sont pas redondantes d'une image à l'autre.

L'activation d'un pixel à partir d'une information asynchrone peut être effectuée en respectant - à une résolution temporelle près - le caractère asynchrone de la séquence d'évènements, de manière à réaliser une activation qui est commandée sur évènement (« event driven »).

Ainsi, le dispositif proposé permet la projection vers des photorécepteurs de l'œil de l'utilisateur (photorécepteurs présents naturellement dans l'œil et/ou photorécepteurs d'un implant visuel) d'un flux lumineux correspondant aux pixels activés de manière asynchrone. L'activation asynchrone des pixels permet de n'activer simultanément qu'un faible nombre de pixels (par exemple un unique pixel ou groupe de pixels colocalisés) et en conséquence de ne stimuler par un flux lumineux qu'une portion locale de la zone de photorécepteurs. L'intensité lumineuse d'un tel flux, ne visant qu'à la stimulation d'une zone localisée, peut alors être portée à des niveaux requis pour l'application envisagée. En particulier, la faible quantité de données représentant une séquence d'images d'un signal asynchrone de type AER permet d'augmenter l'intensité des signaux lumineux d'excitation des photorécepteurs d'une prothèse visuelle ou d'un organe visuel auquel un traitement optogénétique a été appliqué.

Dans un mode de réalisation, l'unité de commande peut en outre être configurée pour, après activation d'un pixel de la matrice à un instant déterminé par un événement indiqué par l'information asynchrone, répéter la commande d'activation dudit pixel sensiblement au même niveau d'activation à des instants définis par une séquence de rafraichissement.

L'activation d'un pixel à partir d'une information asynchrone permet ainsi de ne considérer l'activation d'un pixel que lorsqu'un évènement correspondant à ce pixel est détecté dans les données d'entrée représentant par exemple une séquence d'images, et de n'effectuer des activations de rafraichissement qu'à une fréquence beaucoup plus faible que celle des procédés d'affichage classiques.

Dans un mode de réalisation, la réception de l'information asynchrone peut comprendre la réception d'un signal portant l'information asynchrone, et la commande d'activation d'un pixel comprendre la détection dans le signal d'une information représentant un événement.

De plus, la séquence de rafraîchissement peut définir des instants d'activation du pixel séparés par un intervalle de temps. Cet intervalle de temps entre une activation sur évènement et une activation de rafraîchissement, ou entre deux activations de rafraîchissement, peut par exemple être déterminé en fonction de la persistance rétinienne de l'œil humain. La persistance rétinienne de l'œil humain constitue un seuil limite qu'il est préférable de ne pas dépasser pour effectuer un affichage de rafraîchissement du pixel, au risque de détériorer le confort visuel de l'utilisateur. Par exemple, cet intervalle de temps sera choisi entre 40 ms et 800 ms, et de préférence entre 40 ms et 150 ms, afin d'éviter les effets de scintillement, sachant qu'un intervalle de temps plus long correspond à une fréquence de rafraîchissement moins élevée et une diminution du flux de commandes d'affichage et de calculs associés.

Selon un mode de réalisation du dispositif, le projecteur comprend une matrice de micro-miroirs, une unité de pilotage de la matrice de micro-miroirs, une entrée de commande pour recevoir les commandes d'activation de pixel, et une entrée optique pour recevoir un flux lumineux.

Selon un mode de réalisation du dispositif, le support du projecteur se présente sous la forme d'une paire de lunettes, le projecteur étant placé sur une surface des lunettes.

Selon un autre aspect, il est proposé un système de visualisation d'une scène, comprenant un sous-système de visualisation couplé de manière opérationnelle à un sous-système d'acquisition, dans lequel le sous-système de visualisation comprend un dispositif selon le premier aspect, et dans lequel le sous-système d'acquisition comprend un capteur disposé en regard de la scène, couplé de manière opérationnelle à une unité de traitement configurée pour générer de l'information asynchrone représentant pour chaque pixel des évènements.

Selon un mode de réalisation du système, le capteur est un capteur de lumière comprenant une optique d'acquisition de la scène et une matrice d'éléments photosensibles.

Selon un mode de réalisation du système, le capteur est monté sur le support du projecteur de façon à ce que la scène capturée corresponde sensiblement au champ visuel d'un utilisateur du dispositif.

Selon un mode de réalisation du système, le support du projecteur se présente sous la forme d'une paire de lunettes, le projecteur est monté sur une première surface des lunettes, le capteur est monté sur la partie supérieure de la monture des lunettes, et l'unité de commande du dispositif et l'unité de traitement du sous-système d'acquisition sont montées sur une deuxième surface des lunettes.

En variante, on pourra prévoir un système dans lequel le support du projecteur se présente sous la forme d'une paire de lunettes, le projecteur est monté sur une première surface des lunettes, et le capteur, l'unité de commande du dispositif et l'unité de traitement du sous-système d'acquisition sont montés sur une deuxième surface des lunettes.

D'autres particularités et avantages de la présente invention apparaîtront dans la description ci-après d'exemples de réalisation non limitatifs, en référence aux dessins annexés, dans lesquels :
La figure 1 est un schéma synoptique d'un dispositif de visualisation d'une séquence d'images selon un mode de réalisation du dispositif proposé;
La figure 2 est un schéma synoptique d'un dispositif de projection selon un mode de réalisation du dispositif proposé ;
La figure 3 est un schéma synoptique d'un sous-système optique d'un dispositif de projection selon un mode de réalisation du dispositif proposé ;
Les figures 4a et 4b sont des schémas synoptiques de sous-système optique d'un dispositif de projection selon des modes de réalisation particuliers du dispositif proposé ;
Les figures 5a, 5b, 5c 5d illustrent des paires de lunettes sur lesquelles sont montées un dispositif de visualisation d'une séquence d'images et un capteur de lumière selon des modes de réalisation du dispositif et du système proposés ;
Les figures 6a et 6b sont des diagrammes illustrant des procédés de commande d'activation de pixel mis en œuvre par le dispositif et le système proposés dans un mode de réalisation ;
Les figures 7a et 7b sont des diagrammes montrant une séquence temporelle d'évènements reçus dans un signal asynchrone pour la mise en œuvre du dispositif et du système proposés ;
Les figures 7c et 7d sont des diagrammes montrant une séquence temporelle de commandes d'activation de pixel générées selon des modes de réalisation particuliers du dispositif et du système proposés ;
La figure 8 est un schéma synoptique d'un système de visualisation d'une scène selon un mode de réalisation du système proposé ;
La figure 9a est un schéma synoptique d'un dispositif d'acquisition de la lumière apte à générer un signal asynchrone selon un mode de réalisation du système proposé ;
La figure 9b est un diagramme montrant un exemple de profil d'intensité lumineuse au niveau d'un pixel d'un capteur asynchrone ;
La figure 9c montre un exemple de signal délivré par le capteur asynchrone en réponse au profil intensité de la figure 9b ;
La figure 9d illustre la reconstruction du profil intensité à partir du signal de la figure 9c ;

Dans la description détaillée ci-après de modes de réalisation de l'invention, de nombreux détails spécifiques sont présentés pour apporter une compréhension plus complète. Néanmoins, l'homme du métier peut se rendre compte que des modes de réalisation peuvent être mis en pratique sans ces détails spécifiques. Dans d'autres cas, des caractéristiques bien connues ne sont pas décrites en détail pour éviter de compliquer inutilement la description.

L'invention sera décrite ci-après dans le cadre non limitatif d'une information asynchrone représentant, pour un pixel d'une matrice de pixels, des évènements correspondant à des variations de lumière pour le pixel. Les dispositifs et systèmes proposés ne sont toutefois pas limités à ce mode de réalisation particulier, les évènements concernant le pixel pouvant selon le mode de réalisation correspondre à des variations de lumière pour le pixel, à la détection d'une forme d'intérêt ou à la détection d'une primitive, et plus généralement à tout type d'information asynchrone pour le pixel.

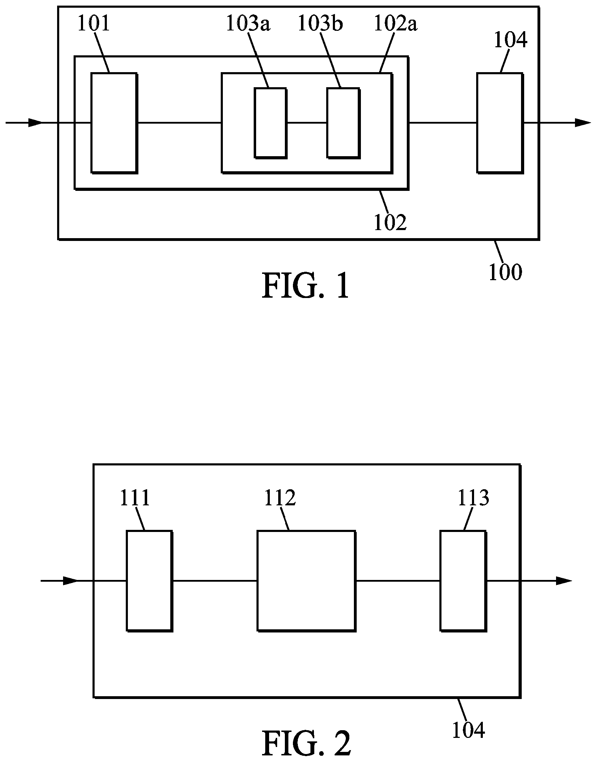

La figure 1 montre un dispositif de visualisation d'une séquence d'images 100 comprenant une unité de commande 102 pour commander l'activation de pixels, et un projecteur 104 pour projeter un flux lumineux sur l'œil d'un utilisateur.

Dans la suite, les images à visualiser sont considérées sous la forme d'une matrice d'objets élémentaires appelés pixels.

Dans un mode de réalisation du dispositif proposé, l'unité de commande 102 comprend une interface d'entrée 101 pour recevoir une information asynchrone. L'information asynchrone reçue sur l'interface d'entrée (101) représente, pour chaque pixel de la matrice, des événements correspondant à des variations de lumière pour le pixel. Elle correspond ainsi à une séquence d'images, chacune considérées sous la forme d'une matrice de pixels.

L'interface d'entrée 101 peut être configurée pour recevoir l'information asynchrone sous différentes formes, ou formats, correspondants à différents modes de réalisation du dispositif. Elle peut en outre être prévue conforme à différents formats standards, tel que, par exemple, le format USB (bus série universel, en anglais Universal Serial Bus). Le dispositif proposé n'est pas limité à un format particulier d'information asynchrone, de vecteur de cette information (par exemple, un signal asynchrone portant de l'information représentative d'un flux d'évènements), ou un format spécifique d'interface d'entrée.

L'information asynchrone peut en effet être générée par différents moyens. Par exemple, elle peut être portée par un signal asynchrone produit par un capteur de vision asynchrone et reçu sur l'interface d'entrée (101). Elle peut aussi résulter de l'encodage d'une séquence d'images de synthèse produisant un ensemble de données reçue sur l'interface (101).

D'une manière générale, l'information asynchrone représente des évènements relatifs aux pixels de la matrice. Dans un mode de réalisation particulier, l'information asynchrone indique, ou signale, des évènements relatifs à un ou plusieurs pixels. Elle peut par exemple comprendre des données qui identifient des évènements avec leurs caractéristiques respectives.

Selon un mode de réalisation, l'information asynchrone pour un pixel peut être portée par une succession d'impulsions ou raies, positives ou négatives, positionnées dans le temps à des instants tₖ dépendant du profil lumineux pour le pixel. Ces raies peuvent être représentées mathématiquement par des pics de Dirac positifs ou négatifs et caractérisées chacune par un instant d'émission tₖ et un bit de signe. L'information correspondant à un évènement pour un pixel, peut comprendre une première information relative à un instant d'occurrence de l'évènement, et une deuxième information relative à une caractéristique lumineuse pour le pixel à cet instant.

La forme de l'information asynchrone pour un pixel peut être différente d'une succession de pics de Dirac, les événements représentés pouvant avoir une largeur temporelle ou une amplitude ou une forme d'onde quelconque.

L'interface d'entrée 101 reçoit ainsi dans ce mode de réalisation, pour chaque pixel de position p = (x ; y), des données représentant une suite d'impulsions binaires, que l'on peut modéliser par des évènements ON ou OFF engendrés de manière asynchrone à des instants respectifs *tₖ*.

L'information correspondant à un événement comprend une information de position du pixel pour lequel l'événement s'est produit (par exemple le couple (x ; y) des numéros de ligne et de colonne dans la matrice de pixels), une information du temps d'occurrence de l'événement (par exemple une valeur discrète de temps par rapport à une référence), et une information de type d'événement (par exemple un bit pour coder des évènements de deux types).

Dans un mode particulier de réalisation, l'interface d'entrée 101 peut être configurée pour recevoir un signal asynchrone portant de l'information représentative d'un flux d'évènements, chacun défini par un quadruplet *e*(*x*; *y*; *t*; *ON*/*OFF*) donnant la position p = (x ; y) du pixel auquel l'évènement est associé, l'instant t auquel l'événement a été détecté, et le type (ON ou OFF) de l'évènement.

Dans un autre mode particulier de réalisation, le signal asynchrone reçu en entrée de l'interface 101 porte de l'information représentative d'un flux d'évènements où chaque évènement est défini par un quadruplet *e*(*x*; *y*; *t*; *g*) donnant la position p = (x ; y) du pixel auquel l'évènement est associé, l'instant t auquel l'évènement a été détecté, et un niveau de gris g associé à l'évènement.

L'unité de commande 102 comprend en outre une unité de traitement des données 102a comportant un processeur 103a couplé de manière opérationnelle à une mémoire 103b ainsi qu'à l'interface d'entrée 101. En fonction du mode de réalisation, la mémoire 103b peut contenir des instructions logicielles qui, lorsqu'elles sont exécutées par le processeur 103a de l'unité de traitement des données 102a, amènent cette unité 102a à effectuer ou contrôler les parties interface d'entrée 101, commande d'activation de pixels selon les différents procédés décrits dans les présentes, et transmission des commandes vers le projecteur 104. L'unité de traitement 102a peut être un composant implémentant un processeur ou une unité de calcul pour la génération de commandes d'activation de pixels selon les différents procédés décrits et le contrôle des interfaces d'entrée 101 et du projecteur 104 du dispositif 100.

En outre, l'interface d'entrée 101, l'unité de traitement des données 102a, le processeur 103a et/ou les moyens mémoire 103b peuvent être, séparément ou conjointement, mis en œuvre sous forme logicielle, comme décrit ci-dessus, sous forme matérielle, comme un circuit intégré spécifique application (ASIC), ou sous forme d'une combinaison d'éléments matériels et logiciels, comme par exemple un ou plusieurs programmes logiciel destinés à être chargés et exécutés respectivement sur un ou plusieurs composants de type FPGA (Field Programmable Gate Array). Ils peuvent être mis en œuvre, séparément ou conjointement, sous forme d'un circuit électronique, ou bien au sein d'un ou de plusieurs composants électroniques (en anglais, chip ou chipset).

De même, différents modes de mise en œuvre du dispositif 100 peuvent être envisagés. Par exemple, l'interface d'entrée 101, l'unité de traitement des données 102a et/ou les moyens mémoire 103b peuvent être implémentés sur un module électronique de traitement d'information asynchrone couplé de manière opérationnelle à un module électronique de projection qui implémente les composants du projecteur 104.

L'unité de commande 102 génère des commandes d'activation de pixel dont le format est adapté à l'interface d'entrée du projecteur 104.

Dans un mode particulier de réalisation du dispositif, l'interface de sortie de l'unité de commande 102 est configurée pour générer un signal de sortie de format adapté pour piloter un projecteur vidéo utilisant la technologie des matrices de micro-miroirs (en anglais DMD, pour « Digital Micromirror Device »). Ce type de projecteur est parfois désigné sous l'acronyme DLP, pour « Digital Light Processing », et fonctionne avec une source de lumière qui vient illuminer une matrice de micro-miroirs qui vibrent en fonction de la quantité de lumière à réfléchir. Les vibrations de chaque miroir s'effectuent autour de deux positions correspondant respectivement à des inclinaisons autour d'un axe, l'une dans laquelle la lumière est réfléchie par le miroir vers une optique de sortie, et l'autre dans laquelle la lumière est réfléchie par le miroir vers une surface absorbante et n'est donc pas projetée. Chaque miroir de la matrice DMD projette de la lumière pour un pixel de la matrice de pixels formant l'image projetée.

Certains projecteurs DLP sont en outre capables d'illuminer des pixels avec différents niveaux de gris, et peuvent donc recevoir des commandes d'activation pour chaque pixel comprenant une information relative à un niveau de gris avec lequel le pixel doit être illuminé. On pourra par exemple utiliser un projecteur DLP capable de gérer 1024 niveaux de gris, et lui fournir des commandes d'activation selon les procédés décrits dans lesquelles le niveau de gris d'illumination d'un pixel est codé sur 10 bits.

La figure 2 montre un exemple de mise en œuvre du projecteur 104 selon un mode particulier de réalisation du dispositif proposé utilisant un projecteur DLP.

Le projecteur 104 comprend une interface d'entrée 111, une unité de pilotage de matrice DMD 112, et un sous-système optique 113 comprenant la matrice DMD.

L'interface d'entrée 111 est couplée de manière opérationnelle à l'unité de commande 102 de la figure 1, et est apte à recevoir des commandes d'activation de pixel générée par l'unité de commande 102. Ce couplage peut être prévu conforme à différents formats standards, tel que, par exemple, le format USB (bus série universel, en anglais Universal Serial Bus).

L'unité de pilotage de matrice DMD pilote le positionnement de chaque miroir de la matrice DMD en fonction des commandes d'activation de pixel reçues par l'interface 111.

La résolution spatiale pour le pilotage des micro-miroirs peut être choisie en fonction de l'application envisagée et de la taille de la matrice de micro-miroirs du DMD utilisé. Par exemple, on peut conserver une résolution spatiale de l'ordre d'un pixel et illuminer un pixel sans activer ses voisins, et donc n'activer qu'un seul micro-miroir. On peut aussi, dans un autre mode de réalisation, regrouper des pixels en paquets, par exemple de 3 x 3 pixels, pour générer différents niveaux de gris en fonction du nombre de pixels activés dans le paquet, et activer les micro-miroirs correspondants.

Par ailleurs, l'interface d'entrée 111 peut être configurée pour recevoir des commandes d'activation de pixel sous différentes formes, ou formats, correspondants à différents modes de réalisation du dispositif. Le dispositif proposé n'est pas limité à un format particulier de commande d'activation de pixel, ou un format spécifique d'interface d'entrée. Le format des commandes d'activation de pixel sera de préférence prévu compatible avec l'interface d'entrée 111 et l'unité de pilotage 112.

Par exemple, les commandes d'activation de pixel reçue par l'interface d'entrée 111 peuvent, dans un mode de réalisation, ne concerner qu'un seul pixel de la matrice, dans la mesure où l'interface d'entrée 111 et l'unité de pilotage 112 sont capables de fonctionner en mode adressage individuel de pixel.

On peut toutefois, dans un autre mode de réalisation du dispositif, utiliser un projecteur qui fonctionne en mode trame, en tirant parti des valeurs élevées (en comparaison avec d'autres types de projecteurs) de résolution temporelle des projecteurs DLP existants (1440 Hz, 4 KHz, 22 KHz). Dans ce mode de fonctionnement, les commandes d'activation comprendront des trames dans lesquelles seuls les pixels objets de la commande d'activation sont allumés.

L'unité de pilotage 112 comprend une unité de traitement des données comportant un processeur couplé de manière opérationnelle à une mémoire (non représentés sur la figure). En fonction du mode de réalisation, la mémoire peut contenir des instructions logicielles qui, lorsqu'elles sont exécutées par le processeur de l'unité de traitement des données, amènent cette unité à effectuer ou contrôler les parties interface d'entrée 111 et pilotage du DMD. L'unité de traitement peut être un composant implémentant un processeur ou une unité de calcul pour la génération de commandes de pilotage du DMD et le contrôle de l'interface d'entrée 111.

En outre, l'interface d'entrée 111 et les différents composants de l'unité de pilotage 112 peuvent être, séparément ou conjointement, mis en œuvre sous forme logicielle, comme décrit ci-dessus, sous forme matérielle, comme un circuit intégré spécifique application (ASIC), ou sous forme d'une combinaison d'éléments matériels et logiciels, comme par exemple un ou plusieurs programmes logiciel destinés à être chargés et exécutés respectivement sur ou plusieurs composants de type FPGA (Field Programmable Gate Array). Ils peuvent être mis en œuvre, séparément ou conjointement, sous forme d'un circuit électronique, ou bien au sein d'un ou de plusieurs composants électroniques (en anglais, chip ou chipset).

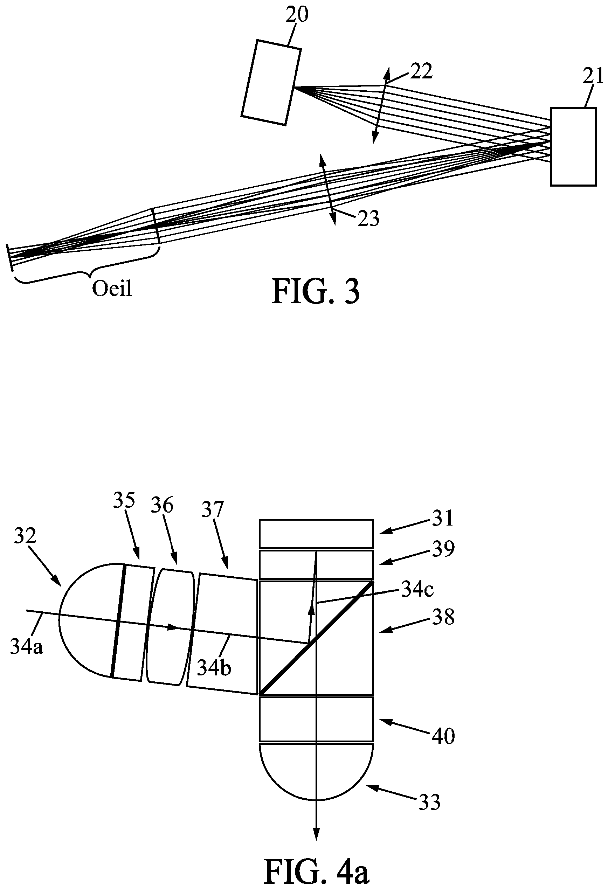

La figure 3 illustre l'architecture du sous-système optique du projecteur 104 selon un mode particulier de réalisation du dispositif proposé.

La figure 3 montre une source lumineuse (20) agencée pour émettre en direction d'une matrice à micro-miroirs (21) un signal optique de projection. Ce signal optique de projection est adapté en fonction de l'application mise en œuvre par le dispositif et des réglementations en vigueur. Par exemple, dans le cas de l'utilisation du dispositif par une personne équipée d'un implant visuel à photodiodes, le signal optique de projection sera adapté pour fournir une intensité lumineuse par pixel qui permette de stimuler les photodiodes de l'implant. De même, dans le cas de l'utilisation du dispositif par une personne ayant bénéficié de traitements optogénétiques, le signal optique de projection sera adapté pour fournir une intensité lumineuse par pixel à des longueurs d'onde spécifiques de cette application qui permette de stimuler la zone traitée. La source lumineuse (20) est donc choisie avec notamment une gamme spectrale et une intensité qui correspond à l'application envisagée. La source lumineuse (20) peut par exemple être une source laser, ou bien une source lumineuse incohérente. Elle est choisie de préférence portative, de manière à ce que l'utilisateur du dispositif proposé puisse aisément transporter le dispositif et l'ensemble des éléments l'accompagnant (source lumineuse, alimentation(s) électrique(s), etc.).

En outre, la source lumineuse (20) peut être déportée et le signal optique de projection qui en est issue être transporté pour illuminer la matrice de micro-miroirs, par exemple par une fibre optique (non représentée sur la figure). Dans ce cas, la source lumineuse (20) peut comprendre une diode laser couplée à une fibre optique pour transporter le signal lumineux vers le composant DMD.

Une première lentille de collimation (22) permet d'obtenir un signal optique de projection focalisé sur l'ensemble de la surface de la matrice de micro-miroirs utilisée. Cette lentille sera donc agencée et positionnée en fonction du composant DMD (21) choisi et de la source lumineuse (20).

Le faisceau lumineux issu de la première lentille (22) vient illuminer la matrice de micro-miroirs, qui est positionnée de manière à ce que les micro-miroirs, positionnés pour réfléchir de la lumière vers une optique de sortie, réfléchissent la lumière selon une direction choisie de manière à illuminer la zone de l'œil dans laquelle sont situés les photodétecteurs que l'on cherche à stimuler (qu'il s'agisse des photodétecteurs présents naturellement, traités ou non par optogénétique, ou de photodétecteurs d'un implant). On choisira de préférence un agencement du sous-système optique qui permet d'illuminer localement la zone fovéale de l'œil.

Dans un mode de réalisation, la matrice de micro-miroirs est positionnée de manière à ce que les micro-miroirs réfléchissent la lumière vers une optique de sortie dans une direction sensiblement alignée sur l'axe optique de l'œil.

Une deuxième lentille de collimation (23) permet de recueillir la lumière réfléchie par un micro-miroir ou un groupe de micro-miroirs co-localisés en un faisceau lumineux de section de dimensions correspondantes à celle de la zone de l'œil à illuminer. De préférence, la deuxième lentille sera agencée de sorte que le faisceau lumineux qui en est issu vienne illuminer localement cette zone de l'œil sur une partie correspondant à la position du micro-miroir ou du groupe de micro-miroirs dans la matrice DMD.

L'optique de l'œil fonctionne en effet comme une lentille de focalisation dont le plan focal objet correspond à la zone fovéale. Le faisceau issu de la deuxième lentille 23 est ainsi focalisé pour stimuler localement la zone fovéale.

On peut par ailleurs prévoir, dans un mode particulier de réalisation, d'adjoindre à la deuxième lentille 23 une lentille de focalisation pour corriger des défauts de vision, comme par exemple une lentille liquide permettant de focaliser sur des objets qui sont situés à une distance pouvant aller d'une dizaine de centimètres à l'infini. Cette lentille liquide peut être commandée par exemple par un potentiomètre permettant à l'utilisateur du dispositif de choisir parmi plusieurs modes de vision, comme par exemple la vision de près, la vision à 1,50 m et la vision de loin.

Dans un mode de réalisation, le faisceau issu de la deuxième lentille 23 est dimensionné de manière à illuminer une zone un peu plus large que la zone fovéale. Le faisceau peut par exemple avoir un diamètre de l'ordre de 4,5 mm permettant d'éviter des pertes d'illuminations de la zone fovéale lorsque l'œil bouge.

La figure 4a illustre un premier mode de réalisation du sous-système optique du projecteur.

On retrouve sur la figure 4a une lentille 32 de collimation combinée à une matrice de lentilles (en anglais, microlens array) 35 pour concentrer la lumière d'un faisceau lumineux 34a issu d'une fibre optique de transport couplée à une source de lumière (non représentés sur la figure), en un signal optique de projection 34b apte à illuminer l'ensemble des micro-miroirs de la matrice de micro-miroirs (DMD) 31 avec les caractéristiques (notamment puissance et longueur d'onde) requises.

La matrice de lentilles permet de limiter la diffraction engendrée par le nombre important de micro-miroirs. Ainsi, dans un mode de réalisation du sous-système optique du projecteur utilisant un composant DMD, une matrice de lentilles peut être insérée sur le chemin lumineux, éventuellement combinée à une modulation réalisée sur la fibre optique, ce afin de réduire l'effet de mouchetage.

Un bloc optique composé d'une lentille 36 et d'un prisme optique 37 permet d'écarter la direction du signal optique de projection d'un angle correspondant à l'angle de pivotement des miroirs du DMD selon leur axe de pivotement. Par exemple, certains composants DMD fonctionnent avec des micro-miroirs qui pivotent autour d'un axe selon un angle de 12°. Ils réfléchissent donc la lumière dans une direction écartée de 12° par rapport à l'axe perpendiculaire à la matrice. L'émission de lumière vers la matrice avec un angle de 12° permet de compenser cet écart.

Le signal optique de projection 34b est ensuite réfléchi sur un miroir dichroïque 38 (en anglais, polarized beam splitter) pour être dirigé vers la matrice de micro-miroirs 31. Une lame quart d'onde 39 (en anglais, quarter wave plates) est placée entre la matrice de micro-miroirs 31 et le miroir dichroïque 38 sur le chemin du signal optique de stimulation 34c issu de la réflexion par la matrice de micro-miroirs 31 pour modifier la polarisation de signal 34c afin qu'il puisse traverser le miroir dichroïque 38 vers l'optique de sortie.

L'optique de sortie comprend une fenêtre optique 40 et une deuxième lentille de collimation 33 jouant un rôle correspondant à celui de la deuxième lentille de collimation 23 de la figure 3.

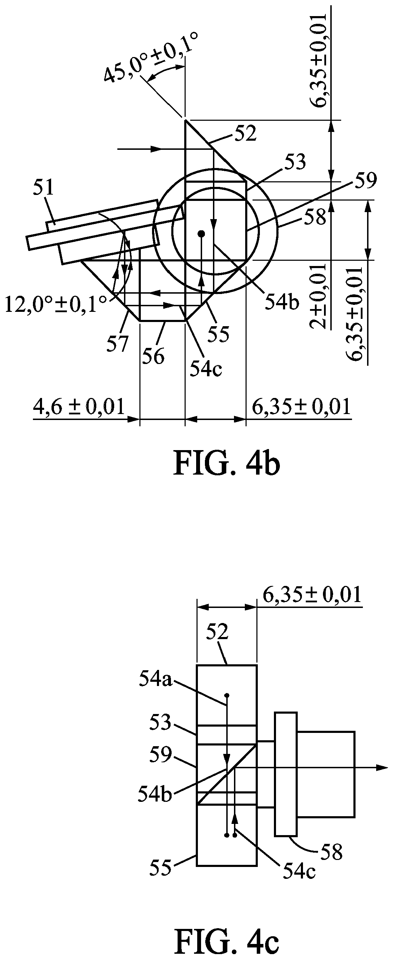

La figure 4b illustre un deuxième mode de réalisation du sous-système optique du projecteur.

Le faisceau lumineux 54a, par exemple généré par une diode laser et transporté par fibre optique vers le sous-système optique du projecteur, est réfléchi par un prisme optique 52 situé en entrée du sous-système optique. On retrouve sur la figure 4b une lentille 53 de collimation, éventuellement combinée à une matrice de lentilles, pour concentrer la lumière du faisceau lumineux 54a issu d'une fibre optique de transport en un signal optique de projection 54b apte à illuminer l'ensemble des micro-miroirs de la matrice de micro-miroirs (DMD) 51 avec les caractéristiques (notamment puissance et longueur d'onde) requises.

Un bloc optique composé d'un miroir dichroïque 59, d'un premier prisme optique 55 séparé d'un deuxième prisme optique 57 par une lentille 56 permet de guider le signal optique de projection 54b vers la matrice de micro-miroirs du composant DMD 51. Comme précédemment, on compense l'écart de projection de introduit par le composant DMD en positionnant ce dernier écarté d'un angle correspondant (dans l'exemple illustré sur la figure, l'écart angulaire est de 12°).

Le chemin du signal optique de stimulation 54c issu de la réflexion par la matrice de micro-miroirs 51 est l'inverse de celui du signal optique de projection 54b de la matrice du composant DMD jusqu'au miroir dichroïque 59, où le signal optique de stimulation 54c est réfléchi vers l'optique de sortie.

L'optique de sortie comprend un bloc fenêtre optique et lentille de collimation 58 jouant un rôle correspondant à celui de la deuxième lentille de collimation 23 de la figure 3.

Les éléments 52, 53, 55, 58 et 59 décrits ci-dessus sont aussi représentés sur la figure 4c où l'on peut voir le trajet du faisceau lumineux 54a et du signal optique de stimulation 54c au sein de ces composants vus sous un autre angle que celui présenté sur la figure 4b.

Les indications de dimension des différents composants indiqués sur la figure 4b illustrent un exemple de réalisation du sous-système optique qui permet d'obtenir un équipement de très petite taille, et suffisamment compact pour être monté sur la face interne d'un verre de lunettes comme décrit ci-après.

Les dimensions et tolérances associées indiquées sur les figures 4b et 4c sont exprimées en mm.

En revenant aux figures 1 et 2, le projecteur 104 est monté sur un support de manière à diriger le signal optique de stimulation issu du sous-système optique du projecteur vers la zone de l'œil que l'on souhaite illuminer.

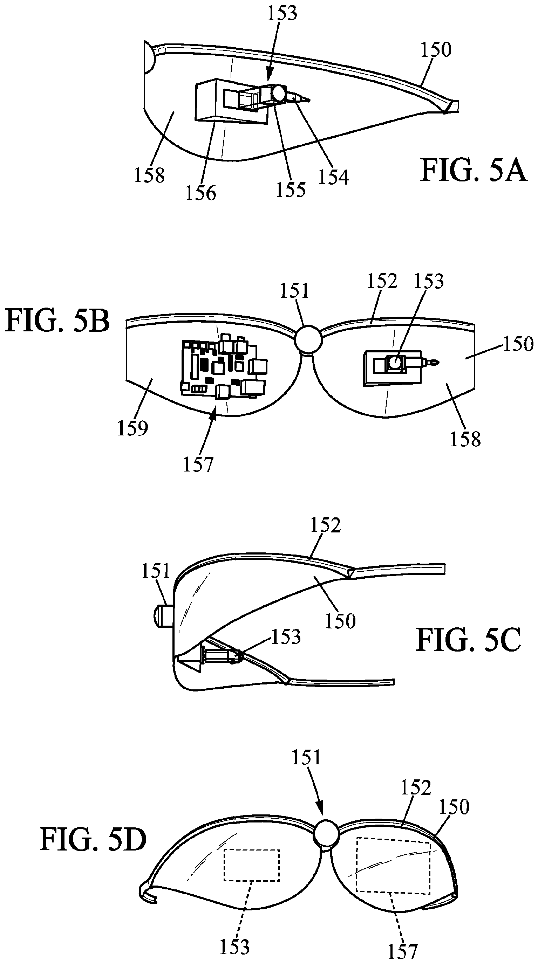

Ce support peut se présenter sous la forme d'une paire de lunettes 150, comme illustré sur les figures 5a, 5b, et 5c, qui montrent un module projecteur 104, 153 monté sur la face intérieure d'un verre d'une paire de lunettes.

Ce support peut aussi se présenter sous la forme d'un masque, ou d'un casque, et sera de préférence conformé pour pouvoir être positionné sur la tête de l'utilisateur, et le projecteur agencé sur le support de manière à illuminer des photorécepteurs de l'œil lors de l'utilisation du dispositif.

La figure 5a montre une paire de lunettes 150 sur laquelle est monté un module projecteur 153. Le module projecteur comprend un sous-système électronique et un sous-système optique qui peuvent être réalisés selon les exemples de mise en œuvre décrits ci-dessus.

Le projecteur 153 illustré sur la figure 5a comprend une interface de couplage de fibre optique 154, une optique de sortie du signal optique de stimulation de photorécepteurs 155, et un bloc optoélectronique 156 comprenant une matrice de micro-miroirs.

Le module projecteur pourra comprendre un projecteur DLP piloté en utilisant les procédés décrits ci-après, qui pourra par exemple comprendre un composant DMD de taille 4,6mm x 5.2mm, et supporter une résolution d'affichage, c'est-à-dire une taille de l'image affichée en pixels, de 608 x 684 pixels (correspondant au standard WVGA) et une résolution temporelle allant jusqu'à 1440 Hz. Ces composants ont une taille suffisamment petite pour permettre le montage du module projecteur sur une paire de lunettes comme illustré sur les figures 5A, 5b et 5c.

On décrit ci-après des procédés de commande d'activation de pixels pouvant être mis en œuvre au sein du dispositif proposé.

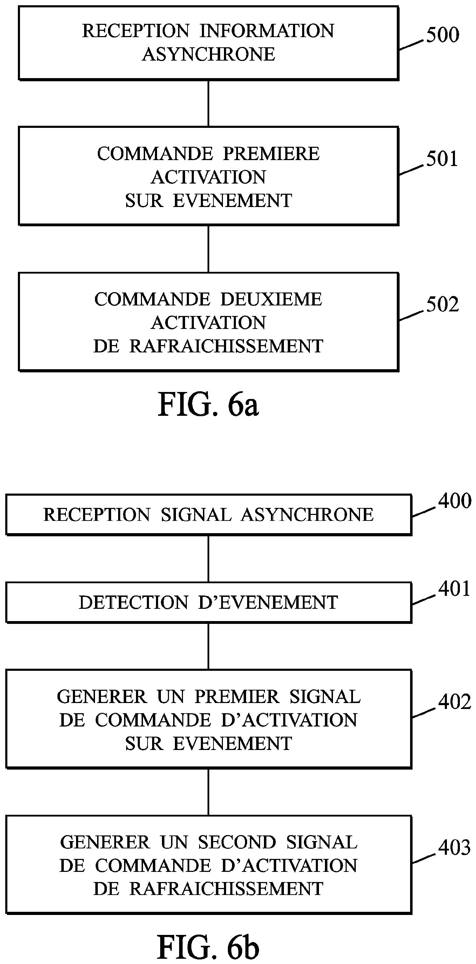

En référence aux figures 1 et 6a, l'unité de commande d'activation de pixels 102 pilote indépendamment chaque pixel d'une matrice de pixels pour l'activation de ce pixel. Le dispositif 100 reçoit (500) par le biais de l'interface d'entrée 101 de l'information asynchrone représentative d'évènements correspondants à des variations de lumière pour le pixel.

Par exemple, pour un pixel de position (xₒ, yₒ) dans la matrice de pixels (pixel positionné sur la ligne d'indice xₒ et sur la colonne d'indice yₒ dans une matrice MxN, avec *x* ∈ {0, *...,M*-1} et *y* ∈ {0, *...,N*-1}, l'information reçue comprendra de l'information asynchrone pour le pixel de position (xₒ, yₒ).

L'information asynchrone est traitée par l'unité de traitement de données 102a pour identifier un évènement pour le pixel afin de commander (501) une première activation du pixel à un instant d'activation déterminé par l'événement identifié.

L'identification d'un évènement pourra viser des évènements caractérisés par une première information indiquant un instant de survenance de l'évènement, et une deuxième information relative à une caractéristique lumineuse pour le pixel à un instant correspondant. Par exemple, l'identification d'un évènement pourra comprendre la détection de deux pics ou impulsions dans un signal portant l'information asynchrone, le premier indiquant un instant de survenance de l'événement et le deuxième un niveau de gris caractéristique de l'événement pour le pixel.

Dans le mode de réalisation dans lequel les évènements sont caractérisés par l'instant de survenance d'une variation d'intensité lumineuse au-delà d'un seuil et le sens de cette variation, la commande d'activation pourra comprendre un niveau d'illumination du pixel déterminé en tenant compte de la variation détectée appliquée au niveau d'illumination de la précédente commande d'activation du pixel.

Dans le mode de réalisation dans lequel les évènements sont caractérisés par l'instant de survenance d'une variation d'intensité lumineuse au-delà d'un seuil et un niveau de gris associé à cette variation, la commande d'activation pourra comprendre un niveau d'illumination du pixel correspondant au niveau de gris déterminé suite à la détection de l'évènement.

Cette première activation du pixel est donc commandée sur identification d'un évènement pour un pixel en utilisant l'information asynchrone reçue en entrée du dispositif de visualisation d'une séquence d'images 100. Dans un mode particulier de réalisation, l'activation est pilotée dès qu'un évènement est identifié pour un pixel, moyennant le temps de traitement nécessaire pour le système 100 pour le traitement des informations relatives à l'évènement. En variante, le système 100 pourra maintenir une référence de temps d'activation, sur la base de laquelle les activations des pixels seront pilotées à des instants correspondant respectivement aux évènements identifiés pour chaque pixel. Comme décrit précédemment, chaque évènement peut être caractérisé par un instant d'occurrence et une ou plusieurs valeurs correspondant à des informations lumineuses respectives (intensité lumineuse, niveau de gris, couleur, etc.).

Dans un mode particulier de réalisation du dispositif proposé, une deuxième activation du pixel pourra être commandée (502) suite à la première activation pour répéter celle-ci à des instants respectifs définis par une séquence de rafraichissement. La première activation peut donc être suivie d'une ou de plusieurs activations destinées à rafraîchir l'activation commandée sur identification d'un événement.

On peut prévoir que la séquence de rafraîchissement définisse des instants d'activation du pixel séparés par un intervalle de temps. Cet intervalle de temps peut être commun à l'ensemble des pixels de la matrice ou bien défini pour chaque pixel ou pour différents sous-ensembles de pixels. Il peut notamment être déterminé en fonction de la persistance rétinienne de l'œil humain. Cela permet de choisir des valeurs d'intervalle de rafraîchissement suffisamment grandes pour éviter l'affichage d'informations redondantes à une fréquence élevée au détriment de l'efficacité du système, tout en tenant compte de la durée de la persistance rétinienne de l'activation précédent. Par exemple, l'intervalle de temps de rafraîchissement peut être choisi entre 40 ms et 150 ms, sachant que plus la valeur choisie sera élevée, plus la commande d'activation de pixels gagnera en efficacité en évitant d'autant plus les activations de redondance.

Une deuxième activation du pixel peut donc être commandée pour effectuer un rafraîchissement de l'activation du pixel pendant l'intervalle de temps ainsi déterminé et courant à partir de l'activation du pixel suite à la précédente commande d'activation.

Cette précédente commande d'activation peut être une commande d'activation sur identification d'événement telle que décrit ci-dessus, ou bien une commande d'activation de rafraîchissement dans le cas par exemple où aucune activation sur identification d'évènement n'a été commandée pendant une durée correspondant à l'intervalle de temps de rafraîchissement courant depuis la précédente activation du pixel.

La figure 6b illustre un mode de réalisation particulier du procédé proposé, dans lequel le dispositif 100 de la figure 1 reçoit un signal portant de l'information asynchrone, traite ce signal pour détecter des évènements, puis génère des commandes d'activation de pixels transmises au projecteur 104 en utilisant un format approprié à ce dernier.

En référence aux figures 1 et 6b, le dispositif de visualisation d'une séquence d'images 100 reçoit (400) par le biais de l'interface d'entrée 101 un signal asynchrone portant de l'information représentative d'évènements correspondants à des variations de lumière pour le pixel. Le signal asynchrone est traité par l'unité de traitement de données 102a pour détecter (401) une information représentant un évènement pour le pixel.

Comme décrit ci-dessus, selon le mode de réalisation du dispositif proposé, la détection d'une information représentant un évènement pourra viser des événements caractérisés par l'instant de survenance d'une variation d'intensité lumineuse au-delà d'un seuil (qui pourra être, dans un mode de réalisation, spécifique au pixel) et le sens de cette variation, ou bien viser des évènements caractérisés par l'instant de survenance d'une variation d'intensité lumineuse au-delà d'un seuil (qui pourra être, dans un mode de réalisation, spécifique au pixel) et un niveau de gris associé à cette variation.

L'information représentant un évènement pour le pixel est traitée par l'unité de traitement de données 102a pour générer (402) un premier signal de commande d'activation du pixel. Le caractère asynchrone du signal d'entrée du dispositif de visualisation d'une séquence d'images 100 sera d'autant mieux respecté que le temps de génération et de transmission d'une commande d'activation du pixel à partir du moment où un évènement est détecté pour le pixel sera court. Un traitement temps-réel sera ainsi préféré afin d'obtenir une activation quasiment instantanée en fonction des évènements détectés pour chaque pixel.

La première commande d'activation, générée sur détection d'un évènement pour un pixel, est transmise au projecteur 104 auquel l'unité de commande 102 est interfacée.

Comme décrit ci-dessus, cette première activation de pixel, effectuée sur détection d'un évènement pour le pixel, pourra être suivie d'une deuxième activation destinée à rafraîchir la précédente activation sur détection d'évènement. Dans ce cas, la deuxième activation donne lieu à la génération (403) d'une deuxième commande d'activation. Cette deuxième commande d'activation est générée de manière à ce que l'activation qui en découle permette le rafraîchissement de l'activation précédemment effectué pour le pixel. Pour ce faire, on détermine un intervalle de temps de rafraichissement dont la durée définit l'écart temporel maximum entre deux activations pour un pixel. Cet intervalle de temps peut être commun à l'ensemble des pixels de la matrice ou bien défini pour chaque pixel ou pour différents sous-ensembles de pixels. Il peut par exemple être déterminé en fonction de la persistance rétinienne de l'œil humain. Cela permet de choisir des valeurs d'intervalle de rafraîchissement suffisamment grandes pour éviter l'affichage d'informations redondantes à une fréquence élevée au détriment de l'efficacité du système, tout en tenant compte de la durée de la persistance rétinienne de la précédente activation.

Un deuxième signal de commande d'activation pourra donc être généré (403) pour effectuer un rafraîchissement de l'activation du pixel pendant l'intervalle de temps ainsi déterminé et courant à partir de l'activation du pixel suite à la précédente commande d'activation. Cette précédente commande d'activation peut être une commande d'activation sur détection d'évènement telle que décrit ci-dessus, ou bien une commande d'activation de rafraîchissement dans le cas par exemple où aucune commande d'activation sur détection d'évènement n'a été générée pendant une durée correspondant à l'intervalle de temps de rafraîchissement courant depuis la précédente activation.

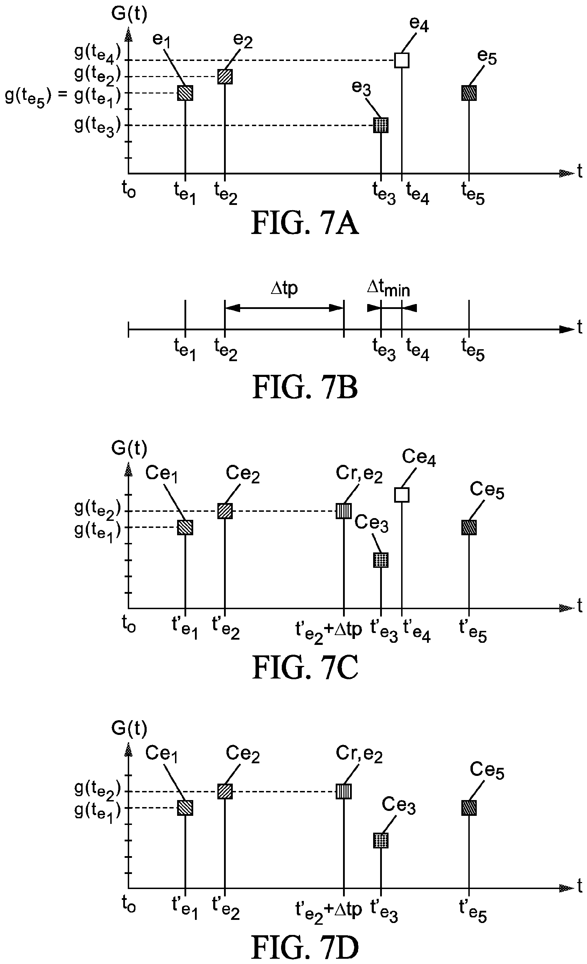

Les figures 7a et 7b illustrent une séquence de signal asynchrone pour un pixel et les commandes d'activation correspondantes.

La figure 7a illustre l'information portée par un échantillon de signal asynchrone dans lequel les évènements pour un pixel sont représentés sur un axe des temps par des pics de Dirac d'amplitude G(t) correspondant à un niveau de gris. Cinq évènements e₁, e₂, e₃, e₄, et e₅ sont représentés sur la figure 7a, positionnés respectivement aux instants te1, te2, te3, te4 et te5 par rapport à une référence de temps t0, avec te₁ < te₂ < te₃ < te₄ < te₅. Chacun des 5 évènements porte une information de niveau de gris pour le pixel considéré notée g(t=teᵢ), avec i = {1, 2, 3, 4, 5}

Ces valeurs de niveau de gris pourront par exemple résulter de la quantification d'une valeur de niveau de gris sur 2*^{n_{quant}}* niveaux et être codés sur n_{quant} bits.

La figure 7b montre une vue simplifiée de la séquence d'évènements illustrée par la figure 7a sur laquelle les évènements sont représentés par des pics de Dirac d'amplitude constante. L'intervalle de temps Δ*tₚ* est défini en fonction de la persistance rétinienne de l'œil et de manière à correspondre à un intervalle de temps maximum séparant deux activations consécutives d'un même pixel. Sur l'exemple illustré sur la figure 7b, les écarts temporels séparant deux évènements consécutifs n'excèdent pas la quantité Δ*tₚ*, à l'exception de l'écart entre les évènements e₂ et e₃ te₃ - te₂.

Dans l'exemple d'une séquence d'évènements illustrée sur les figures 7a et 7b, on pourra générer une commande d'activation du pixel suite à la détection de l'évènement e₁ pour une activation avec les caractéristiques (typiquement un niveau de gris) portées par le signal asynchrone traité pour l'évènement e₁. Il en est de même des évènements e₂, e₃, e₄, et e₅ dont les détections, dans un mode de réalisation, pourront chacune donner lieu à la génération d'une commande d'activation avec des caractéristiques respectives.

Dans un mode particulier de réalisation du procédé proposé, une commande d'activation sera en outre générée pour effectuer une activation de rafraîchissement dans un intervalle de temps prédéterminé suite à l'activation du pixel sur détection de l'évènement e₂. Cette commande d'activation pourra par exemple être générée si aucun évènement n'a été détecté dans l'intervalle de temps [te₂ ; te₂+Δ*tₚ*] de durée Δ*tₚ* à partir de l'instant te₂.

En variante elle pourra être générée à tout instant pendant la durée de l'intervalle de temps [te₂ ; te₂+Δ*tₚ*] de durée Δ*tₚ* à partir de l'instant te₂.

La figure 7c montre un exemple de séquence de commandes d'activation générées selon un mode de réalisation du procédé proposé appliqué à la séquence d'évènements de la figure 7a. Les commandes d'activation sont représentées sur la figure par des pics de Dirac d'amplitude variable. Elle montre des commandes d'activations Ce₁, Ce₂, Ce₃, Ce₄ et Ce₅, correspondant respectivement aux évènements e₁, e₂, e₃, e₄ et e₅ de la figure 7a, générées respectivement à des instants tce₁, tce₂, tce₃, tce₄ et tce₅. Chacune des commandes comporte une information relative au niveau de gris auquel le pixel doit être illuminé, notée sur la figure g(teᵢ), avec i = {1, 2, 3, 4, 5}.

Outre les commandes d'activation Ceᵢ générées sur détection d'un évènement eᵢ, une commande d'activation de rafraîchissement Cr,e₂ est générée à l'instant t'e₂+Δ*tₚ* suite à la non-détection d'un nouvel évènement consécutif à celle de l'évènement e₂ pendant une durée Δ*tₚ*.

La commande d'activation de rafraichissement Cr,e₂ pourra être générée avec les mêmes caractéristiques d'activation, et par exemple le même niveau de gris g(te₂), que celles comprises dans la commande d'activation sur évènement Ce₂. En variante, la commande d'activation de rafraichissement Cr,e₂ pourra être générée avec des caractéristiques d'activation déterminées en fonction de celles déterminées pour la commande d'activation sur évènement Ce₂.

Le rafraîchissement des pixels de la matrice peut aussi être effectué en groupe, et il peut être procédé à un rafraichissement systématique et périodique de pixels de la matrice à une fréquence de rafraîchissement prédéterminée. L'avantage de ce mode de réalisation est d'éviter une gestion individualisée de chaque pixel pour ce qui concerne le rafraichissement. Le rafraichissement de la matrice est alors répété à une cadence au moins égale à l'intervalle de rafraichissement le plus court défini pour un pixel.

Selon cette variante, le rafraichissement de la matrice de pixels est effectué périodiquement, tout en restant complètement décorrélé et indépendant de la séquence de commandes d'activation sur événement. La génération de la commande de rafraichissement d'activation est indépendante de celle de la commande d'activation sur événement, et la fréquence de rafraichissement d'activation est choisie de manière à ce que l'écart entre une activation sur évènement de chaque pixel et l'activation de rafraichissement immédiatement subséquente n'excède pas un intervalle de temps prédéterminé Δ*tₚ* qui, comme discuté ci-dessus, pourra être choisi en fonction de la persistance rétinienne de l'œil humain de l'ordre de 40ms à 150ms. Ce mode « mixte » d'activation permet de combiner une activation sur évènement fonctionnant de manière asynchrone avec une activation périodique de rafraichissement effectué de manière synchrone. Ainsi, en fonction de la survenance des évènements pour chaque pixel, l'activation de rafraichissement du pixel pourra survenir dans un très court laps de temps suivant une activation sur évènement du pixel, ou bien à l'issue d'une durée prédéterminée suivant une activation sur évènement du pixel définie comme l'écart maximum entre deux activations du pixel. Ce mode mixte permet d'alléger la gestion du rafraichissement de l'activation, tout en limitant grâce à l'activation sur évènement asynchrone pour chaque pixel, l'activation de l'information de redondance, étant donné que l'activation asynchrone permet de choisir des fréquences de rafraichissement faibles en comparaison des systèmes actuels.

En référence à la figure 1, chaque génération de commande d'activation sur évènement pourra par exemple donner lieu à la mise en mémoire 103b par l'unité de traitement de données 102a de caractéristiques d'activation relatives à la commande générée, de sorte que ces caractéristiques puissent être récupérées par l'unité 102 pour la gestion de l'activation de rafraichissement.

La figure 7d représente schématiquement une suite de commandes d'activation générées selon un autre mode de réalisation du procédé proposé appliqué à la séquence d'évènements des figures 7a et 7b.

En référence à la figure 7b, l'évènement e4 est détecté avec un espacement temporel te₄ - te₃ inférieur à l'intervalle de temps Δ*ₜₘᵢₙ* défini comme l'intervalle de temps minimum séparant deux commandes d'activation sur évènement successives du pixel.

La séquence de commandes d'activation de la figure 7d diffère de la séquence illustrée par la figure 7c en ce qu'aucune commande d'activation n'est générée pour l'évènement e₄, étant donné que l'écart temporel entre l'instant de détection de cet évènement et l'instant de détection de l'évènement immédiatement précédent est inférieur à un seuil prédéfini Δ*ₜₘᵢₙ.*

La gestion de l'intervalle de temps de séparation pour chaque pixel peut être réalisée à l'aide d'un temporisateur, réglé à la valeur Δ*ₜₘᵢₙ* définie pour le pixel et déclenché à chaque génération de commande d'activation sur évènement pour le pixel. Par exemple, un évènement détecté alors que ce temporisateur n'a pas expiré pour le pixel pourra conduire à ignorer l'évènement et ne pas générer de commande d'activation sur évènement correspondante.

Les caractéristiques d'activation portées par l'information asynchrone pour cet évènement ignoré pourront être aussi ignorées. En variante, on pourra prévoir une mise en œuvre selon laquelle, quand bien même aucune commande d'activation sur évènement n'est générée, les caractéristiques d'activation correspondant à l'évènement sont consignées en mémoire 103b pour être utilisées ultérieurement, par exemple pour la prochaine activation de rafraichissement du pixel.

Selon le mode de réalisation, un seul temporisateur peut être utilisé pour la gestion de l'intervalle de rafraichissement et celle de l'intervalle de séparation. Ce temporisateur peut être déclenché à chaque génération de commande d'activation pour le pixel, en consignant en mémoire un indicateur permettant de distinguer les commandes d'activation sur évènement des commandes d'activation de rafraichissement.

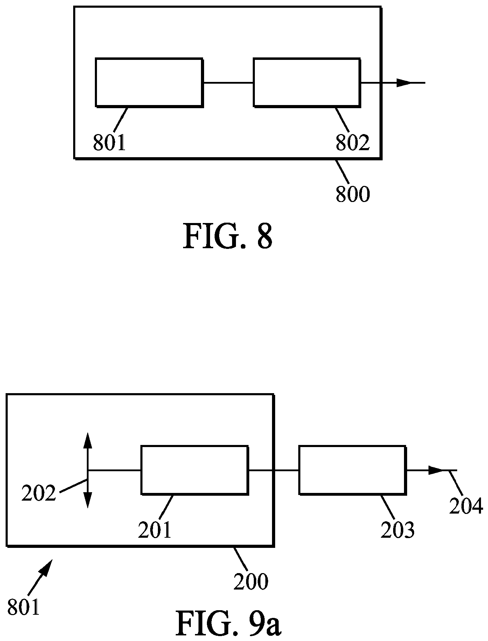

La figure 8 montre un système de visualisation d'une scène 800 comprenant un sous-système d'acquisition 801 et un sous-système de visualisation 802.

Le sous-système d'acquisition 801 comprend un dispositif d'acquisition de la lumière apte à générer un signal portant de l'information asynchrone représentant, pour chaque pixel d'une matrice de pixels, des évènements correspondant respectivement à des variations de lumière dans la scène. Il est couplé de manière opérationnelle au sous-système de visualisation 802, qui comprend un dispositif de visualisation tel que décrit ci-dessus comprenant une interface d'entrée configurée pour recevoir un signal portant de l'information asynchrone.

Le signal portant de l'information asynchrone transitant sur l'interface de couplage entre les sous-systèmes 801 et 802 peut avoir différentes formes, ou formats, correspondants à différents modes de réalisation du système. L'interface de couplage peut par ailleurs être prévue conforme à différents formats standards, tel que, par exemple, le format USB. Le système proposé n'est pas limité à un format particulier d'information asynchrone, de vecteur de cette information (par exemple, un signal asynchrone portant de l'information représentative d'un flux d'évènements), ou un format spécifique d'interface de couplage entre les sous-systèmes 801 et 802.

Le signal asynchrone reçu par le sous-système 802 porte de l'information représentative d'évènements temporels correspondant à des variations de lumière dans une scène. A cet effet, le sous-système 802 peut comprendre une interface d'entrée configurée pour recevoir un signal produit par le sous-système d'acquisition 801.

La figure 9a montre un exemple de mise en œuvre du sous-système d'acquisition 801 selon un mode particulier de réalisation du système de visualisation proposé.

La figure 9a montre un sous-système d'acquisition 801 comprenant un dispositif d'acquisition de la lumière 200 comprenant un capteur de vision asynchrone basé sur événements (201) placé en regard d'une scène et recevant le flux lumineux de la scène à travers une optique d'acquisition (202). Le capteur (201) peut comporter un groupement d'éléments photosensibles organisés en une matrice de pixels, de sorte que chaque pixel de la matrice corresponde à un élément photosensible du capteur. Pour chaque pixel de la matrice, le dispositif (200) engendre une séquence de signal asynchrone basé sur évènement à partir des variations de lumière ressenties par le pixel dans la scène apparaissant dans le champ de vision du dispositif (200). Chaque pixel correspondant à un élément photosensible, produit ainsi des évènements temporels correspondant respectivement à des variations de lumière dans la scène.

Le capteur 201 ne produit donc pas des trames vidéo constituées par la matrice de pixels correspondant aux éléments photosensibles du capteur à une fréquence d'échantillonnage prédéterminée. Il réagit pour chaque pixel de la matrice à des évènements correspondant à des variations de lumière pour le pixel. A l'inverse, il ne produit pas d'information pour un pixel si aucun évènement ne se produit pour ce pixel. Il n'effectue en particulier pas de capture systématique d'intensité lumineuse des pixels de la matrice. Ainsi, les évènements auxquels il réagit sont asynchrones, et ne dépendent pas d'une fréquence d'acquisition de trames vidéo. Cela permet de diminuer fortement, si ce n'est supprimer, les redondances créées par l'acquisition de trames vidéo à un rythme déterminé ne tenant pas compte de l'absence de changement de l'information portée par un pixel d'une trame à l'autre.

Une unité de traitement (203) traite l'information issue du capteur (201) et représentative des évènements produits de manière asynchrone par les différents pixels, pour générer un signal asynchrone portant cette information.

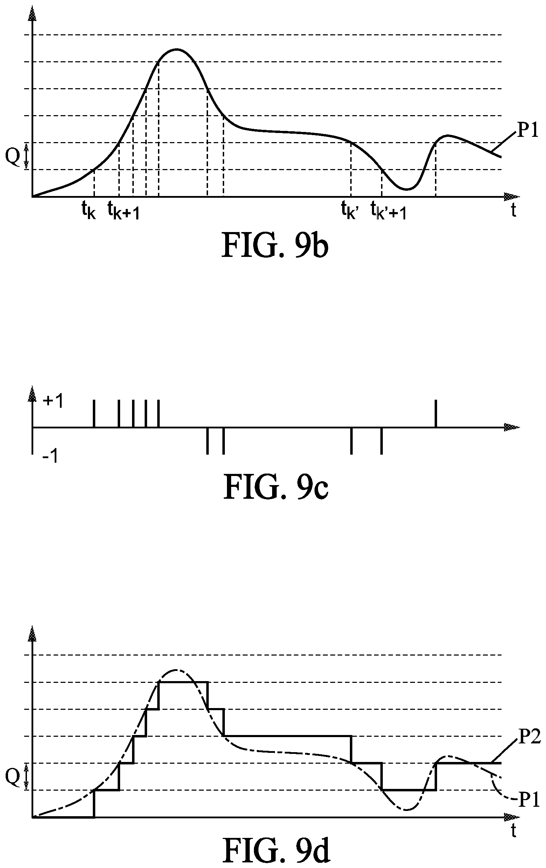

Un exemple de principe d'acquisition par ce capteur asynchrone est illustré par les figures 9b-9d. Selon cet exemple, l'information consiste en une succession d'instants, notés *tₖ* (*k* = 0,1,2,...) auxquels un seuil d'activation Q est atteint. Le capteur 201 est donc muni d'un détecteur de variations qui, pour chaque pixel, mesure et enregistre l'intensité lumineuse du pixel lorsque cette intensité a varié au-delà d'un seuil Q.

La figure 9b montre un exemple de profil d'intensité lumineuse P1 vue par un pixel de la matrice du capteur de vision asynchrone. Chaque fois que cette intensité augmente d'une quantité égale au seuil d'activation Q en comparaison de ce qu'elle était à l'instant tₖ, un nouvel évènement est identifié et une raie positive (niveau +1 sur la figure 9c) est émise correspondant à l'instant de dépassement du seuil différentiel Q, noté tₖ₊₁. Symétriquement, chaque fois que l'intensité du pixel diminue de la quantité Q en comparaison de ce qu'elle était à l'instant t_{k'}, un nouvel évènement est identifié et une raie négative (niveau -1 sur la figure 9c) est émise correspondant à l'instant de dépassement du seuil différentiel Q, noté t_{k'+1}.

La figure 9d montre le profil d'intensité P2 qu'on est capable de reconstruire comme une approximation du profil P1 par intégration dans le temps du signal asynchrone de la figure 9c.

Le seuil d'activation Q peut être fixe, comme dans le cas des figures 9b-d, ou adaptatif en fonction de l'intensité lumineuse. Par exemple, le seuil ±Q peut être comparé aux variations du logarithme de l'intensité lumineuse pour la génération d'un événement ±1.

La classe de capteurs photosensibles asynchrones générant des évènements à partir des variations d'intensité lumineuse est désignée par l'acronyme DVS, pour « Dynamique Vision Sensor » (capteur de vision dynamique).

À titre d'exemple, le capteur 201 peut être un capteur DVS du genre décrit dans "A 128x128 120 dB 15 µs Latency Asynchronous Temporal Contrast Vision Sensor", P. Lichtsteiner, et al., IEEE Journal of Solid-State Circuits, Vol. 43, No. 2, février 2008, pp. 566-576, ou dans la demande de brevet US 2008/0135731 A1. Un capteur DVS utilisé pour un dispositif d'aide à la vision est décrit dans le brevet FR 2 975 251.

Une autre génération de capteurs photosensibles asynchrones permet de générer de l'information asynchrone indiquant des événements ainsi qu'une caractéristique associée, par exemple un niveau de gris.

L'article de Posch, C., Matolin, D., et Wohlgenannt, R. (2011) intitulé "A qvga 143 db dynamic range frame-free pwm image sensor with lossless pixel-level video compression and time-domain cds", et publié dans le IEEE Journal of Solid-State Circuits, 46, pages 259 -275. doi:10.1109/JSSC.2010.2085952, fournit une description d'exemples d'évènements codés par des niveaux de gris.

L'information asynchrone pour chaque pixel consiste là-encore en une succession d'impulsions ou raies positionnées dans le temps aux instants *tₖ* dépendant du profil lumineux pour le pixel. Chaque évènement peut par exemple correspondre à deux impulsions successives, la première indiquant l'instant de l'évènement et la seconde permettant de déterminer un niveau de gris pour le pixel en fonction de l'écart temporel entre les deux impulsions. L'information correspondant à un évènement pour un pixel comprend ainsi une première information relative à un instant d'occurrence de l'évènement, et une deuxième information relative à une caractéristique lumineuse (niveau de gris) pour le pixel à cet instant.

Le sous-système d'acquisition 801 peut incorporer dans un mode de réalisation un capteur de vision asynchrone basé sur évènements 201, de nouvelle génération, que l'on désigne parfois sous l'acronyme ATIS, pour « Asynchronous, Time-Based Image Sensor ». Le sous-système d'acquisition et le capteur ATIS qu'il incorpore peuvent être par exemple du type de celui décrit dans l'article de C. Posch et al., intitulé « An Asynchronous Time-based Image Sensor » (IEEE International Symposium on Circuits and Systems, 2008, pages 2130-2133), ou bien du type de celui décrit dans l'article de C. Posch et al., intitulé « A QVGA 143 dB dynamic range frame-free PWM image sensor with lossless pixel-level video compression and time-domain CDS » (46(1) :259275, 2011).

La dynamique de la rétine (durée minimum entre les potentiels d'action) de l'ordre de quelques millisecondes peut être convenablement reproduite avec un capteur du type DVS ou ATIS. La performance en dynamique est en tout cas largement supérieure à celle qu'on peut atteindre avec une caméra vidéo classique ayant une fréquence d'échantillonnage réaliste. Par exemple, un capteur de ce type permet d'atteindre des résolutions temporelles de l'ordre de la microseconde avec une gamme de luminance supérieure à 120 dB, ce qui est très supérieur à une caméra standard CMOS/CCD qui possède typiquement une gamme de luminance de 60-70 dB.

Dans un mode de réalisation, l'unité de traitement 203 comprend un processeur couplé de manière opérationnelle à une mémoire. La mémoire peut contenir des instructions logicielles qui, lorsqu'elles sont exécutées par le processeur de l'unité de traitement des données amènent cette unité à traiter les signaux reçus du capteur et générer l'information asynchrone représentant pour chaque pixel des évènements correspondant à des variations de lumière concernant le pixel selon les différents procédés décrits dans les présentes, et transmission de l'information asynchrone sur une interface de sortie. L'unité de traitement peut être un composant implémentant un processeur ou une unité de calcul pour la génération de l'information asynchrone selon les différents procédés décrits et le contrôle du capteur de vision asynchrone du dispositif 200 au sein du sous-système 801.

En outre, l'unité de traitement, et en particulier son processeur et/ou ses moyens mémoire peuvent être, séparément ou conjointement, mis en œuvre sous forme logicielle, comme décrit ci-dessus, sous forme matérielle, comme un circuit intégré spécifique application (ASIC), ou sous forme d'une combinaison d'éléments matériels et logiciels, comme par exemple un ou plusieurs programmes logiciel destinés à être chargés et exécutés respectivement sur ou plusieurs composants de type FPGA (Field Programmable Gate Array). Ils peuvent être mis en œuvre, séparément ou conjointement, sous forme d'un circuit électronique, ou bien au sein d'un ou de plusieurs composants électroniques (en anglais, chip ou chipset).

En référence aux figures 5b, 5c, et 5d, le capteur de vision asynchrone peut être monté sur le support sur lequel le projecteur est monté. Les figures 5b, 5c, et 5d représentent l'exemple de réalisation dans lequel le support est une paire de lunettes 150. Le capteur 151 est monté sur la partie supérieure 152 de la monture des lunettes. De préférence, le capteur sera monté centré sur la partie supérieure 152 des lunettes, de manière à éviter les erreurs de parallaxe entre la scène acquise et celle reproduite par stimulation de photorécepteurs d'un œil par le projecteur 153.

En variante, on peut prévoir de disposer le capteur sur une surface des lunettes, telle que la surface - interne ou externe - d'un des verres des lunettes 150.

Par « verre », on entend une partie des lunettes de forme surfacique fixée sous la partie supérieure des lunettes, et qui peut être constituée de verre mais aussi de tout autre matériau, non nécessairement translucide. Une paire de lunettes comprend deux verres, chaque verre étant formé d'une surface interne et d'une surface externe.

Lorsque le projecteur 153 est monté sur une première surface 158 des lunettes 150, l'unité de commande du dispositif et l'unité de traitement du sous-système d'acquisition peuvent être montées sur une deuxième surface 159 des lunettes. Ces deux unités peuvent être mise en œuvre sur un module électronique 157 fixé sur la surface interne d'un verre des lunettes, le projecteur 153 étant fixé sur la surface interne de l'autre verre, comme illustré sur les figures 5b et 5d.

En variante, un bloc optoélectronique constitué du capteur, de l'unité de commande du dispositif et de l'unité de traitement du sous-système d'acquisition peut être monté sur une surface d'un des verres des lunettes, le projecteur étant monté sur une surface de l'autre verre ou sur l'autre surface du même verre.

Bien que décrits à travers un certain nombre d'exemples de réalisation détaillés, le procédé de commande d'activation et l'équipement pour la mise en œuvre du procédé comprennent différentes variantes, modifications et perfectionnements qui apparaîtront de façon évidente à l'homme de l'art, étant entendu que ces différentes variantes, modifications et perfectionnements font partie de la portée de l'invention, telle que définie par les revendications qui suivent.

De plus, différents aspects et caractéristiques décrits ci-dessus peuvent être mis en œuvre ensemble, ou séparément, ou bien substitués les uns aux autres, et l'ensemble des différentes combinaisons et sous combinaisons des aspects et caractéristiques font partie de la portée de l'invention. En outre, il se peut que certains systèmes et équipements décrits ci-dessus n'incorporent pas la totalité des modules et fonctions décrits pour les modes de réalisation préférés.

Les informations et signaux décrits dans le présent document peuvent être représentés selon une multitude de technologies et de techniques. Par exemple, les instructions, messages, données, commandes, informations, signaux, bits et symboles peuvent être représentés par des tensions, intensités, ondes électromagnétiques ou une combinaison de ces derniers.

En fonction du mode de réalisation choisi, certains actes, actions, évènements ou fonctions de chacune des méthodes décrites dans le présent document peuvent être effectués ou se produire selon un ordre différent de celui dans lequel ils ont été décrits, ou peuvent être ajoutés, fusionnés ou bien ne pas être effectués ou ne pas se produire, selon le cas. En outre, dans certains modes de réalisation, certains actes, actions ou évènements sont effectués ou se produisent concurremment et non pas successivement.

## Revendications

1. Dispositif de visualisation d'une séquence d'images sous forme d'une matrice de pixels, comprenant une unité de commande (102) couplée de manière opérationnelle à un projecteur (104), l'unité de commande (102) comprenant
∘ une interface d'entrée (101) configurée pour recevoir de l'information asynchrone représentant, pour chaque pixel de la matrice, des évènements concernant le pixel ; et
∘ un processeur (103a) configuré pour commander l'activation de chaque pixel de la matrice à des instants déterminés par des événements respectifs indiqués par l'information asynchrone pour ledit pixel ;
dans lequel le projecteur (104) est agencé sur un support (150) de manière à illuminer des photorécepteurs de l'œil lors de l'utilisation du dispositif, et configuré pour projeter un flux lumineux correspondant aux pixels activés par l'unité de commande (102).

2. Dispositif selon la revendication 1, dans lequel l'unité de commande est en outre configurée pour, après activation d'un pixel de la matrice à un instant déterminé par un événement indiqué par l'information asynchrone, répéter la commande d'activation dudit pixel sensiblement au même niveau d'activation à des instants définis par une séquence de rafraichissement.

3. Dispositif selon la revendication 1 ou 2, dans lequel le projecteur comprend une matrice de micro-miroirs, une unité de pilotage de la matrice de micro-miroirs, une entrée de commande pour recevoir les commandes d'activation de pixel, et une entrée optique pour recevoir un flux lumineux.

4. Dispositif selon l'une quelconque des revendications précédentes, dans lequel le support du projecteur se présente sous la forme d'une paire de lunettes, le projecteur étant placé sur une surface des lunettes.

5. Système de visualisation d'une scène, comprenant un sous-système de visualisation (802) couplé de manière opérationnelle à un sous-système d'acquisition (801), dans lequel :
• le sous-système de visualisation (802) comprend un dispositif selon l'une quelconque des revendications 1 à 4 ;
• le sous-système d'acquisition (801) comprend un capteur disposé en regard de la scène, couplé de manière opérationnelle à une unité de traitement (203) configurée pour générer de l'information asynchrone représentant des évènements pour chaque pixel.

6. Système selon la revendication 5, dans lequel le capteur est un capteur de lumière comprenant une optique d'acquisition de la scène et une matrice d'éléments photosensibles.

7. Système selon la revendication 5 ou 6, dans lequel le capteur est monté sur le support du projecteur de façon à ce que la scène capturée corresponde sensiblement au champ visuel d'un utilisateur du dispositif.

8. Système selon l'une quelconque des revendications 5 à 7, dans lequel :
• le support du projecteur se présente sous la forme d'une paire de lunettes,
• le projecteur est monté sur une première surface des lunettes ;
• le capteur est monté sur la partie supérieure de la monture des lunettes ;
• l'unité de commande du dispositif et l'unité de traitement du sous-système d'acquisition sont montées sur une deuxième surface des lunettes.

9. Système selon l'une quelconque des revendications 5 à 7, dans lequel :
• le support du projecteur se présente sous la forme d'une paire de lunettes,
• le projecteur est monté sur une première surface des lunettes ;
• le capteur, l'unité de commande du dispositif et l'unité de traitement du sous-système d'acquisition sont montés sur une deuxième surface des lunettes.

## Patentansprüche

1. Vorrichtung zur Anzeige einer Bildsequenz in Form einer Matrix aus Pixeln, aufweisend eine Steuereinheit (102), die operativ mit einem Projektor (104) gekoppelt ist, wobei die Steuereinheit (102) aufweist:
* eine Eingangsschnittstelle (101) zum Empfangen von asynchroner Information, die für jedes Pixel der Matrix Ereignisse, die das Pixel betreffen, repräsentiert; und
* einen Prozessor (103a) zur Steuerung der Aktivierung jedes Pixels der Matrix an Zeitpunkten, die durch jeweilige Ereignisse bestimmt sind, die von der asynchronen Information für dieses Pixel angegeben sind;
in welcher der Projektor (104) auf einem Träger (150) angeordnet ist, derart, um bei der Verwendung der Vorrichtung Lichtrezeptoren des Auges zu beleuchten, und konfiguriert ist, einen Lichtstrom, der den von der Steuereinheit (102) aktivierten Pixeln entspricht, zu projizieren.

2. Vorrichtung nach Anspruch 1, in welcher die Steuereinheit ferner konfiguriert ist, um nach einer Aktivierung eines Pixels der Matrix an einem Zeitpunkt, der durch ein von der asynchronen Information angegebenes Ereignis bestimmt ist, den Befehl zur Aktivierung des Pixels im Wesentlichen mit dem gleichen Aktivierungspegel an Zeitpunkten, die durch eine Auffrischungssequenz definiert sind, zu wiederholen.

3. Vorrichtung nach Anspruch 1 oder 2, in welcher der Projektor aufweist: eine Matrix aus Mikrospiegeln, eine Einheit zur Ansteuerung der Mikrospiegel-Matrix, einen Steuereingang zum Empfangen der Pixelaktivierungsbefehle und einen optischen Eingang zum Empfangen eines Lichtstroms.

4. Vorrichtung nach einem der vorstehenden Ansprüche, in welcher der Träger des Projektors in Form einer Brille ist und der Projektor auf einer Oberfläche der Brille angeordnet ist.

5. System zur Anzeige einer Szene, aufweisend ein Anzeige-Subsystem (802), das operativ mit einem Erfassung-Subsystem (801) gekoppelt ist,
in welchem:
* das Anzeige-Subsystem (802) eine Vorrichtung nach einem der Ansprüche 1 bis 4 aufweist;
* das Erfassung-Subsystem (801) einen gegenüber der Szene angeordneten Sensor aufweist, der operativ mit einer Verarbeitungseinheit (203) gekoppelt ist, die konfiguriert ist, um asynchrone Information zu generieren, die Ereignisse für jedes Pixel repräsentiert.

6. System nach Anspruch 5, in welchem der Sensor ein Lichtsensor ist, der eine Erfassungsoptik zur Erfassung der Szene und eine Matrix aus lichtempfindlichen Elementen aufweist.

7. System nach Anspruch 5 oder 6, in welchem der Sensor an dem Träger des Projektors angebracht ist, derart, dass die erfasste Szene im Wesentlichen dem Gesichtsfeld eines Anwenders der Vorrichtung entspricht.

8. System nach einem der Ansprüche 5 bis 7, in welchem:
* der Träger des Projektors in Form einer Brille ist,
* der Projektor an einer ersten Oberfläche der Brille angebracht ist;
* der Sensor an dem oberen Teil des Brillengestells angebracht ist;
* die Steuereinheit der Vorrichtung und die Verarbeitungseinheit des Erfassung-Subsystems an einer zweiten Oberfläche der Brille angebracht sind.

9. System nach einem der Ansprüche 5 bis 7, in welchem:
* der Träger des Projektors in Form einer Brille ist,
* der Projektor an einer ersten Oberfläche der Brille angebracht ist;
* der Sensor, die Steuereinheit der Vorrichtung und die Verarbeitungseinheit des Erfassung-Subsystems an einer zweiten Oberfläche der Brille angebracht sind.

## Claims

1. Device for displaying a sequence of images in the form of a matrix of pixels, comprising a control unit (102) coupled operationally to a projector (104), the control unit (102) comprising
- an input interface (101) configured so as to receive asynchronous information representing, for each pixel in the matrix, events concerning the pixel; and
- a processor (103a) configured to control activation of each pixel in the matrix at instants determined by respective events indicated by the asynchronous information for said pixel;
in which the projector (104) is arranged on a support (150) so as to illuminate photoreceptors of the eye during the use of a device, and configured to project a light stream relative to the pixels activated by the control unit (102).

2. Device according to claim 1, in which the control unit is further configured so as, after activation of a pixel in the matrix at an instant determined by an event indicated by the asynchronous information, to repeat the command for activation of said pixel substantially at the same activation level at instants defined by a refresh sequence.

3. Device according to claim 1 or 2, in which the projector comprises a matrix of micromirrors, a unit controlling the matrix of micromirrors, a control input for receiving the pixel-activation commands, and an optical input for receiving a light stream.

4. Device according to any of the preceding claims, in which the projector support is in the form of a pair of spectacles, the projector being placed on a surface of the spectacles.

5. System for displaying a scene, comprising a display sub-system (802) coupled operationally to an acquisition sub-system (801), in which:
- the display sub-system (802) comprises a device according to any of claims 1 to 4;
- the acquisition sub-system (801) comprises a sensor disposed opposite the scene, coupled operationally to a processing unit (203) configured so as to generate asynchronous information representing events for each pixel.

6. System according to claim 5, in which the sensor is a light sensor comprising a optic for acquiring the scene and a matrix of photosensitive elements.

7. System according to claim 5 or 6, in which the sensor is mounted on the projector support so that the captured scene corresponds substantially to the visual field of a user of the device.

8. System according to any of claims 5 to 7, in which:
- the projector support is in the form of a pair of spectacles,
- the projector is mounted on a first surface of the spectacles;
- the sensor is mounted on a top part of the spectacles frame;
- the control unit of the device and the processing unit of the acquisition sub-system are mounted on a second surface of the spectacles.

9. System according to any of claims 5 to 7, in which:
- the projector support is in the form of a pair of spectacles,
- the projector is mounted on a first surface of the spectacles;
- the sensor, the control unit of the device and the processing unit of the acquisition sub-system are mounted on a second surface of the spectacles.
